# EUROPÄISCHE PATENTANMELDUNG

(11) **EP 3 037 804 A1**
(43) Veröffentlichungstag der Anmeldung: **29.06.2016**
(21) Anmeldenummer: 14199823.7
(22) Anmeldetag: 22.12.2014
(51) Int. Cl.: G01N 15/14, G01N 15/02, G01N 21/15, G01N 21/958, G06K 9/40, G01N 15/00

(54) **Verfahren zum quantitativen und qualitativen Erfassen von Partikeln in Flüssigkeit**

(71) Anmelder: Grundfos Holding A/S, 8850 Bjerringbro (DK)
(72) Erfinder: Iversen, Kåre, 8870 Langå (DK); Guldbæk Smith, Christian, 8541 Skødstrup (DK); Dahlqvist, Mathis, 7100 Vejle (DK)
(74) Vertreter: Patentanwälte Vollmann & Hemmer

(57) **Zusammenfassung**

Die Erfindung betrifft ein Verfahren zum quantitativen und/oder qualitativen Erfassen von Partikeln in Flüssigkeit, bei dem die zu untersuchende Flüssigkeit in einen Strahlengang einer optischen Einrichtung zwischen mindestens einer Lichtquelle und einem Bilderfassungssensor mit einer Matrix von lichtempfindlichen Zellen angeordnet wird, bei dem die Pixelwerte der Zellen erfasst und die Verteilung der Pixelwerte zumindest teilweise ermittelt wird, und bei dem der oder die Pixelwerte, die am häufigsten ermittelt worden sind als Wert oder Durchschnittswert für ein Hintergrundsignal verwendet werden, wobei beim Erreichen eines vorbestimmten Grenzwertes für das Hintergrundsignal ein Signal abgegeben oder das Verfahren unterbrochen wird.

## Beschreibung

Die Erfindung betrifft ein Verfahren zum Erfassen von Partikeln in Flüssigkeit mit den im Oberbegriff des Anspruchs 1 angegebenen Merkmalen.

Verfahren zum optischen Erfassen von Partikeln in einer Flüssigkeit zählen zum Stand der Technik. Es wird in diesem Zusammenhang auf EP 2 469 264 A1, WO 2010/063293 A1 und WO 2014/094790 A1 verwiesen, bei denen die Erfassung mittels eines Bilderfassungssensors mit einer Matrix von lichtempfindlichen Zellen, beispielsweise eines CCD-Sensors erfolgt, bei dem die zu untersuchende Flüssigkeit in den Strahlengang einer optischen Einrichtung zwischen mindestens einer Lichtquelle und dem Bilderfassungssensor eingeführt ist. Diese Vorrichtungen sind zwar zur qualitativen und quantitativen Bestimmung von Partikeln in einer Flüssigkeit im stationären Zustand bestimmt, werden jedoch auch für quasi stationäre kontinuierliche Untersuchungen, beispielsweise zur regelmäßigen Überprüfung von Trinkwasser, genutzt. Sie haben hierzu in der Regel einen Probenträger, dessen Flüssigkeit austauschbar ist. Hierzu sind entsprechende Ventile, gegebenenfalls Pumpen und eine Ablaufsteuerung vorgesehen.

Bei der Auswertung des Signals des Bilderfassungssensors zählt es zum Stand der Technik die Pixelwerte der einzelnen Zellen zu erfassen und die statistische Verteilung der Pixelwerte zumindest teilweise zu ermitteln. Der Pixelwert oder die Pixelwerte, die dabei am häufigsten ermittelt werden, werden als Wert oder Durchschnittswert für ein Hintergrundsignal verwendet, welches bei der späteren Signalaufbereitung vom eigentlichen Pixelwert subtrahiert wird, um auf diese Weise rechnerisch den Hintergrund bzw. das Hintergrundrauschen auszublenden und die Partikelerfassung zu vereinfachen und zu verbessern. Es wird in diesem Zusammenhang auf Kim, Seongjai und Lim, Hyeona "Method of Background Subtraction for Medical Image Segmentation" in Nature 2013 verwiesen.

Insbesondere bei der Untersuchung von Trinkwasser aber auch bei anderen Flüssigkeiten, die eine Anreicherung von Ablagerungen oder organisches Wachstum, wie beispielsweise Biofilm, begünstigen, ist die Langzeitstabilität des Probenträgers, insbesondere des Probenträgerfensters ein Problem. Die Ablagerungen reichern sich nicht plötzlich sondern kontinuierlich und das mit nicht vorhersehbarer Geschwindigkeit an und führen dazu, dass die Ergebnisse im Laufe der Zeit immer ungenauer werden bis hin zum vollständigen Versagen der Messung. Es ist daher erforderlich, dass der Probenträger, d. h. das Bauteil, in welchem die Flüssigkeit längs eines Fensters geführt wird, welches im Strahlengang der optischen Einrichtung angeordnet ist, in regelmäßigen Abständen ausgetauscht wird. Da die Geschwindigkeit der Zunahme der Ablagerungen variiert, muss der Austausch des Probenträgers vorsorglich in vergleichsweise kurzen Abständen erfolgen, was aufwändig und teuer ist.

Vor diesem Hintergrund liegt der Erfindung die Aufgabe zugrunde, ein Verfahren zu schaffen, welches diese Ablagerungen erfasst und das nach Möglichkeit mit vorhandenen Mitteln.

Diese Aufgabe wird mit dem Verfahren gelöst, wie es in Anspruch 1 angegeben ist. Eine entsprechend ausgestaltete Vorrichtung zur Ausführung dieses Verfahrens ist in Anspruch 12 angegeben. Vorteilhafte Ausgestaltungen der Erfindung ergeben sich aus den Unteran-sprüchen, der nachfolgenden Beschreibung und der Zeichnung. Dabei können die in den Unteransprüchen und der Beschreibung angegebenen Merkmale jeweils für sich aber auch in geeigneter Kombination die erfindungsgemäße Lösung gemäß Anspruch 1 bzw. Anspruch 12 weiter ausgestalten.

Bei dem erfindungsgemäßen Verfahren zum quantitativen und/oder qualitativen Erfassen von Partikeln in Flüssigkeit wird die zu untersuchende Flüssigkeit in einen Strahlengang einer optischen Einrichtung zwischen mindestens einer Lichtquelle und einem Bilderfassungssensor mit einer Matrix von lichtempfindlichen Zellen angeordnet. Dabei werden die Pixelwerte der Zellen erfasst und die Verteilung der Pixelwerte zumindest teilweise ermittelt, wobei der oder die Pixelwerte, die am häufigsten ermittelt worden sind, als Wert oder Durchschnittswert für ein Hintergrundsignal verwendet werden, wie dies grundsätzlich zum Stand der Technik zählt. Gemäß der Erfindung wird jedoch beim Erreichen eines vorbestimmten Grenzwertes für das Hintergrundsignal ein Signal abgegeben oder das Erfassungsverfahren unterbrochen.

Grundgedanke des erfindungsgemäßen Verfahrens ist es, den an sich ohnehin zu bestimmenden Wert für das Hintergrundsignal dahingehend auszuwerten, ob dieser unterhalb oder über einem vorbestimmten Grenzwert für das Hintergrundsignal liegt, wobei bei Erreichen dieses Grenzwertes oder beim Überschreiten ein Signal abgegeben wird, oder das Verfahren unterbrochen wird. Es wird also ein ohnehin zu bestimmender Wert dafür ermittelt, um festzustellen, ob ein unzulässig hoher Ablagerungs- bzw. Verschmutzungsgrad des Untersuchungsfensters aber auch der übrigen Optik zwischen Lichtquelle und Sensor vorliegt, um entweder ein Signal abzugeben, welches das Erreichen oder Überschreiten dieses Grenzwertes signalisiert oder das Erfassungsverfahren zu unterbrechen. Dabei kann, wenn der Grenzwert mit ausreichendem Abstand zu einem möglichen maximal zulässigen Wert angegeben wird, eine Signalabgabe ausreichen, welche den Anwender davon in Kenntnis setzt, dass nun in Kürze der Probenträger auszuwechseln oder zumindest zu reinigen ist oder eine automatische Steuerung hierzu in Gang setzt.

Das erfindungsgemäße Verfahren zum quantitativen und/oder qualitativen Erfassen von Partikeln in Flüssigkeit ist grundsätzlich unabhängig davon, ob die Flüssigkeit ruht oder strömt. Bevorzugt wird das erfindungsgemäße Verfahren jedoch für quasi stationäre Flüssigkeiten angewendet, das heißt die zu untersuchende Flüssigkeit wird in einem Probenträger einströmen gelassen, wonach jedoch Zu- und Ablauf verschlossen und eine gewisse Zeit gewartet wird, bis die in der Flüssigkeit befindlichen Partikel sich nicht mehr bewegen, das heißt bei schwebenden Partikeln einen quasi stationären Zustand erreicht haben und bei Partikeln, die deutlich schwerer oder leichter als die Flüssigkeit sind, diese entweder vollständig abgesunken oder aufgeschwommen sind.

Das erfindungsgemäße Verfahren hat den Vorteil, dass es vorhandene Ressourcen nutzt und quasi softwaremäßig implementierbar ist, das heißt keine zusätzlichen Kosten verursacht.

Idealerweise wird dabei die Verteilung der Pixelwerte von der gesamten Matrix von lichtempfindlichen Zellen ermittelt. Es versteht sich, dass es auch genügen kann, wenn lediglich ein Großteil dieser Matrix ausgewertet wird, wenn z. B. ein Teil der Zellen zu anderen Zwecken genutzt werden soll und hierfür nicht zur Verfügung steht. Es muss dabei nicht notwendigerweise ein Pixelwert sein, der am häufigsten ermittelt worden ist, sondern es können auch mehrere Pixelwerte sein. Üblicherweise ergibt sich in einem Verteilungsdiagramm ein Peak, der dann als Wert oder Durchschnittswert für ein Hintergrundsignal verwendet wird, beispielsweise durch Bildung eines Mittelwertes. Dieses Hintergrundsignal, welches auch als Rauschsignal bezeichnet wird, wird bei der späteren Auswertung der Pixelwerte von jedem einzelnen Pixelwert abgezogen, um so eine quasi rauschfreie Auswertung zu gewährleisten.

Dabei können gemäß einer vorteilhaften Weiterbildung der Erfindung bei Erreichen eines ersten vorbestimmten Grenzwertes für das Hintergrundsignal ein Signal abgegeben und bei Erreichen eines zweiten vorbestimmten Grenzwertes das Erfassungsverfahren unterbrochen werden. Eine solche zweistufige Lösung ist sinnvoll, um frühzeitig auf eine zunehmende Verschmutzung der optischen Einrichtung, insbesondere des Probenträgerfensters hinzuweisen und in einem weiteren Schritt spätestens dann, wenn die Verschmutzung so hoch ist, dass eine statistisch abgesicherte Auswertung nicht mehr möglich ist, das Erfassungsverfahren zu stoppen. Die Grenzwerte sind dabei zweckmäßigerweise so vorzubestimmen, dass der erste Grenzwert deutlich unterhalb der zulässigen Grenze liegt, innerhalb der eine zuverlässige quantitative und vorzugsweise auch qualitative Erfassung von Partikeln in der Flüssigkeit gewährleistet ist und der zweite Grenzwert gerade unterhalb dieses Grenzwertes liegt oder durch den Grenzwert selbst gebildet ist, sodass bei Erreichen dieses Grenzwertes sichergestellt ist, dass keine Erfassung mehr erfolgt bzw. die Auswertung abgebrochen wird.

Bei dem erfindungsgemäßen Verfahren werden vorteilhaft eine Vielzahl von Bildern in unterschiedlichen Bildebenen aufgenommen, so wie dies beispielsweise in WO 2014/094790 A1 beschrieben ist. Um hinsichtlich des Hintergrundsignals eine statistisch abgesicherte Auswertung vorzunehmen, ist es zweckmäßig, das Hintergrundsignal durch den Mittelwert von Werten oder Durchschnittswerten mehrerer nacheinander aufgenommener Bilder zu bilden, vorzugsweise von 500 bis 1500 Bildern unterschiedlicher Bildebenen, jedoch derselben Flüssigkeitsprobe.

Um nicht nur die Qualität der aktuellen Untersuchung sicherzustellen, sondern insbesondere die Zunahme von Ablagerungen oder Verschmutzungen innerhalb der optischen Einrichtung, insbesondere des Probenträgerfensters zu erfassen und auszuwerten, ist gemäß einer vorteilhaften Weiterbildung des erfindungsgemäßen Verfahrens vorgesehen, dass die Werte für das Hintergrundsignal von zeitlich nacheinander erfolgenden Erfassungen von Partikeln in Flüssigkeit registriert und anhand der zeitlichen Veränderung der registrierten Werte eine Änderungsgeschwindigkeit bestimmt wird. Anhand dieser Änderungsgeschwindigkeit ist dann ohne weiteres bestimmbar, wie lange der Erfassungsprozess noch fortgesetzt werden kann, ohne dass ein Eingriff in das System, sei es zu Reinigungszwecken oder zum Austausch von Bauteilen erforderlich ist. Dabei gibt die Änderungsgeschwindigkeit Hinweis darauf, mit welcher Zunahme von Ablagerungen und Verunreinigungen in welcher Zeit zu rechnen sein wird. Auch gestattet die Ermittlung der Änderungsgeschwindigkeit frühzeitig zu erkennen, wenn unerwartete und plötzliche Änderungen innerhalb des optischen Systems eintreten.

Gemäß einer vorteilhaften Weiterbildung des erfindungsgemäßen Verfahrens kann die Registrierung dieser Werte für das Hintergrundsignal und die Bestimmung der zeitlichen Veränderung der registrierten Werte vorteilhaft dazu genutzt werden, zu bestimmen, wann ein vorbestimmter Grenzwert für das Hintergrundsignal voraussichtlich erreicht sein wird. Eine entsprechend ausgestattete Vorrichtung kann also in einer entsprechenden Anzeige angeben, wann eine Reinigung der Vorrichtung oder ein Wechsel des Probenträgers voraussichtlich spätestens zu erfolgen hat.

Das Hintergrundsignal wird also zweckmäßigerweise als Maß für die Verunreinigungen im Strahlengang herangezogen und bei Erreichen eines vorbestimmten Grenzwertes ein die zu untersuchende Flüssigkeit aufnehmender Flüssigkeits- bzw. Probenträger gereinigt oder gewechselt, das heißt durch einen neuen oder gereinigten Flüssigkeitsträger ersetzt.

Da die Verschmutzung in solchen optischen Einrichtungen, insbesondere die hier in Rede stehende Biofilmbildung innerhalb des Probenträgerfensters in der Regel nicht schlagartig, sondern über mehrere Tage oder Wochen hinweg erfolgt, ist grundsätzlich eine ständige Überprüfung dieses Hintergrundsignals nicht erforderlich. Gemäß der Erfindung wird die Bestimmung des Hintergrundsignals jedoch vorteilhaft vor jedem Erfassen von Partikeln oder zumindest in vorbestimmten zeitlichen Abständen erfolgen, um eine ausreichende Systemsicherheit zu gewährleisten. Gemäß einer vorteilhaften Weiterbildung der Erfindung kann es grundsätzlich auch vorgesehen sein, den Verschmutzungsgrad des Probenträgers in unbefülltem Zustand zu ermitteln, d. h. das Hintergrundsignal zu bestimmen, wenn der Probenträger noch nicht mit Flüssigkeit gefüllt ist bzw. diesen vor dem Messvorgang zu entleeren und vorzugsweise auch zu trocknen.

Dabei kann gemäß einer vorteilhaften Weiterbildung der Erfindung bei der Auswertung der Pixelwerte der Zellenmatrix des Sensors diese Matrix in eine Vielzahl von vorzugsweise gleich großen Teilmatrizen erfolgen, und für jede Teilmatrix ein Hintergrundsignal gebildet werden. Dabei wird die Teilmatrix oder werden die Teilmatrizen, deren Hintergrundsignal einen vorbestimmten weiteren Grenzwert übersteigt, von der weiteren Auswertung zum Erfassen der Partikel ausgenommen. Diese erfindungsgemäße Weiterbildung hat den Vorteil, dass einzelne stark verschmutzte Teile des Probenträgerfensters gezielt bei der Erfassung ausgenommen werden, hierzu zählen auch größere Partikel, wie beispielsweise Luftblasen oder Partikel in dieser Größenordnung, wenn es beispielsweise um die Erfassung von Partikeln geht, die deutlich kleiner sind, wie beispielsweise Bakterien oder Trübstoffe.

Die Verteilung der Pixelwerte kann nicht nur zur Ermittlung des Hintergrundsignals verwendet werden, vorteilhaft werden damit auch die Partikel größenmäßig kategorisiert. Insbesondere wenn es auf die Erfassung von bestimmten Partikeln ankommt, kann eine solche größenmäßige Kategorisierung schon eine erste Auswahl sein, die verfahrensmäßig und auch auswertetechnisch relativ einfach durchgeführt werden kann.

Die Auswertung der Pixelwerte erfolgt zweckmäßigerweise digital. Unabhängig davon hat es sich als zweckmäßig erwiesen, den Grenzwert für das Hintergrundsignal und/oder den weiteren Grenzwert für das Hintergrundsignal einer Teilmatrix auf 10 bis 20% eines maximalen Pixelwertes vorzubestimmen. Diese Prozentangabe ist unabhängig von der Auflösung des Pixelwertes. Bei einer 8 Bit Auflösung eines Pixelwertes, was einer Auflösung von 256 Helligkeitsstufen entspricht, hat es sich als vorteilhaft erwiesen, den Grenzwert für das Hintergrundsignal zwischen 30 und 50 festzulegen, vorzugsweise zwischen 35 und 40 vorzubestimmen. Dies bedeutet also, dass bei der vorgegebenen Auflösung nach Abzug des Hintergrundsignals noch in jedem Falle mehr als 200 unterschiedliche Helligkeitsstufen verbleiben. Dabei ist dieser Grenzwert unabhängig davon, ob es sich um eine optische Einrichtung handelt, bei welcher die Lichtquelle das Probenträgerfenster direkt durchstrahlt (Bright-Field Technique) oder, ob es sich um eine solche handelt, bei welcher nur indirektes Licht den Probenträger erreicht (Dark-Field Technique). Wesentlich ist, dass in beiden Fällen jeweils ein Messbereich von mehr als 200 Stufen verbleibt.

Als weiteren Grenzwert für das Hintergrundsignal einer Teilmatrix hat sich bei einer 8 Bit Auflösung ein Wert zwischen 35 und 45 als vorteilhaft erwiesen. Es werden also bei dem erfindungsgemäßen Verfahren dann nur die Teilmatrizen vom weiteren Erfassungsvorgang ausgenommen, welche ein Hintergrundsignal der Teilmatrix aufweisen, welche einen Wert zwischen 35 und 45 übersteigen, je nachdem welcher Wert hier vorbestimmt worden ist.

Die erfindungsgemäße Vorrichtung zum optischen Erfassen von Partikeln in einer Flüssigkeit weist eine Lichtquelle, einen Bilderfassungssensor mit einer Matrix von lichtempfindlichen Zellen und einen im Strahlengang dazwischen angeordneten Flüssigkeitsträger auf, sowie eine Steuer- und Auswerteelektronik, welche die Pixelwerte der Zellen erfasst und die Verteilung der Pixelwerte zumindest teilweise ermittelt und die von dem oder den Pixelwerten, die am häufigsten ermittelt worden sind, als Wert oder Durchschnittswert für ein Hintergrundsignal verwendet wird. Gemäß der Erfindung ist die Steuer- und Auswerteelektronik so ausgestaltet, dass beim Erreichen eines vorbestimmten Grenzwertes für das Hintergrundsignal ein Signal abgegeben oder der Erfassungsvorgang unterbrochen wird. In analoger Weise kann die Steuer- und Auswerteelektronik gemäß der Erfindung ausgebildet werden, um auch die vorbeschriebenen weiteren Verfahrensmerkmale auszuführen.

Teile der Erfindung sind nachfolgend anhand von Darstellungen erläutert, die folgendes zeigen:
Fig. 1 ein Diagramm, welches zum einen den Verlauf des Hintergrundsignals über einen Zeitraum von mehr als einem Jahr und zum anderen die Häufigkeitsverteilungen der Pixelwerte für diesen Zeitraum darstellt.
Fig. 2a ein mittels eines CCD-Sensors erfasstes Bild einer Probe,
Fig. 2b die Häufigkeitsverteilung der Pixelwerte des Bildes gemäß Fig. 2a,
Fig. 3a ein weiteres Bild entsprechend Fig. 2a bei erhöhter Ablagerungsbildung,
Fig. 3b die Häufigkeitsverteilung der Pixelwerte des Bildes gemäß Fig. 3a,
Fig. 4a in stark vereinfachter Darstellung ein Bild mit großen und kleinen Partikeln,
Fig. 4b wie die großen Partikel des Bildes 4a bei der Auswertung ausgeblendet werden,
Fig. 5a ein weiteres Bild entsprechend Fig. 4a, und
Fig. 5b die entsprechenden Ausblendungen bei der Auswertung.

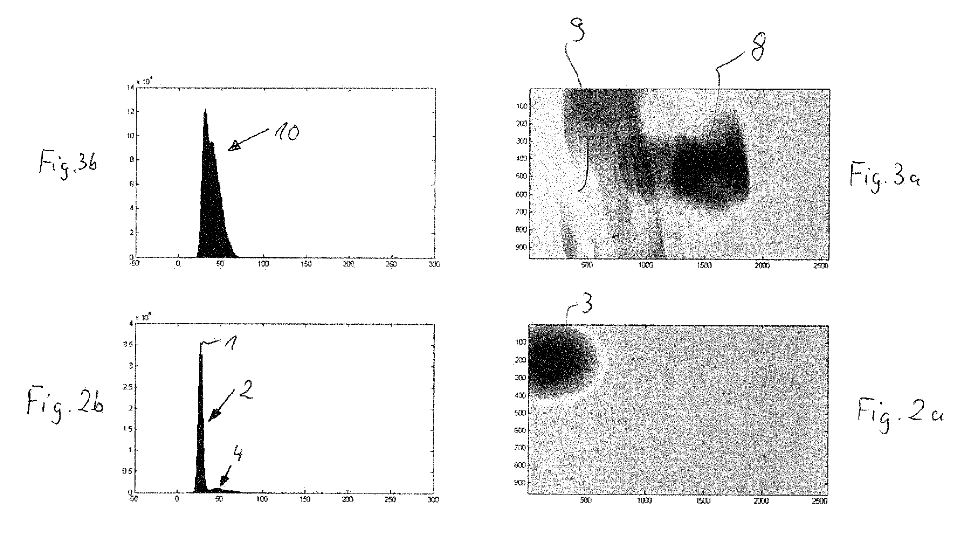

Bei der quantitativen und qualitativen Erfassung von Partikeln in Flüssigkeit wird eine an sich bekannte Vorrichtung eingesetzt, bei welcher ein Probenträger mit einem Fenster zwischen einer Lichtquelle und einem CCD-Sensor angeordnet ist. Dabei kann die Beleuchtung des Fensters entweder direkt erfolgen (Bright-Field Technique) oder indirekt (Dark-Field Technique), was jedoch für die hier in Rede stehenden Verfahren nicht von Bedeutung ist. Die Darstellungen gemäß den Fig. 2a bis 5a sind, wie dies zur Untersuchung von Trinkwasser zweckmäßig ist, in Dark-Field Technique aufgenommen, jedoch invertiert dargestellt, insbesondere um die Vervielfältigung und die optische Erkennbarkeit sicherzustellen. Tatsächlich erscheinen die erfassten Partikel in Dark-Field Technique weiß vor schwarzem bzw. grauem Hintergrund. Sowohl bei Anwendung der Bright-Field Technique als auch bei Anwendung der Dark-Field Technique wird zunächst vor der eigentlichen Signalauswertung des Sensors ein Wert für ein Hintergrundsignal bestimmt. Dies erfolgt anhand von einer Vielzahl von Einzelbildern, im Folgenden ist dies jedoch aus Gründen der besseren Übersicht nur anhand eines Bildes jeweils dargestellt.

Es wird also beispielsweise zu dem Bild gemäß Fig. 2a eine Häufigkeitsverteilung der Pixelwerte ermittelt. Der CCD-Sensor mit dem das Bild gemäß Fig. 2a erstellt worden ist, weist eine Matrix von lichtempfindlichen Zellen auf, wobei jede Zelle entsprechend der zugeführten Lichtmenge elektrische Ladungen erzeugt, die als elektrisches Signal ausgegeben werden, wobei für jede Zelle ein Pixelwert ermittelt wird, der dem Ladungswert der Zelle entspricht. Bei der hier gewählten Auflösung von 8 Bit kann somit jede Zelle 256 Helligkeitswerte ermitteln, vom hellsten Weiß bis zum tiefsten Schwarz.

Dabei wird zunächst für das Bild gemäß Fig. 2a ermittelt, welche Helligkeitswerte, d. h. welche Pixelwerte in welcher Anzahl in der Matrix vorkommen. Das Ergebnis ist in dem Diagramm nach Fig. 2b wiedergegeben. Der Pixelwert 1, der in dem Bild gemäß Fig. 2a am meisten vorkommt, hat eine Helligkeit von 25 bei einer Häufigkeit von 3,5 x 10⁵, wie in Fig. 2b zu erkennen ist. Unmittelbar benachbart sind weitere Helligkeitswerte, die eine ähnlich hohe Häufigkeit aufweisen, und zwar zwischen 20 und 30. In diesem Bereich ist der Peak 2 der Fig. 2b angeordnet, welcher den hellgrauen Bereich des Bildes gemäß Fig. 2a repräsentiert. Aus diesem Peak 2 wird ein Mittelwert gebildet, der das Hintergrundsignal bildet und die hellgraue Fläche in Fig. 2a repräsentiert.

Dieses Hintergrundsignal ist bei der Bildauswertung hinderlich, da es das gesamte Bild überstrahlt. Es wird daher bei der späteren Bildauswertung von jedem einzelnen Pixelwert abgezogen, sodass für die Auswertung ein Bild zur Verfügung steht, welches im Idealfall keinen grauen, sondern einen weißen (tatsächlich schwarzen) Hintergrund hat. Bei der Dark-Field Technique erfolgt dies in umgekehrter Weise, sodass ein im Idealfall tiefschwarzer Hintergrund gebildet ist, wobei sich die Partikel, nicht wie in Fig. 2a in dunkler Farbe, sondern in heller Farbe vor diesem schwarzen Hintergrund abheben.

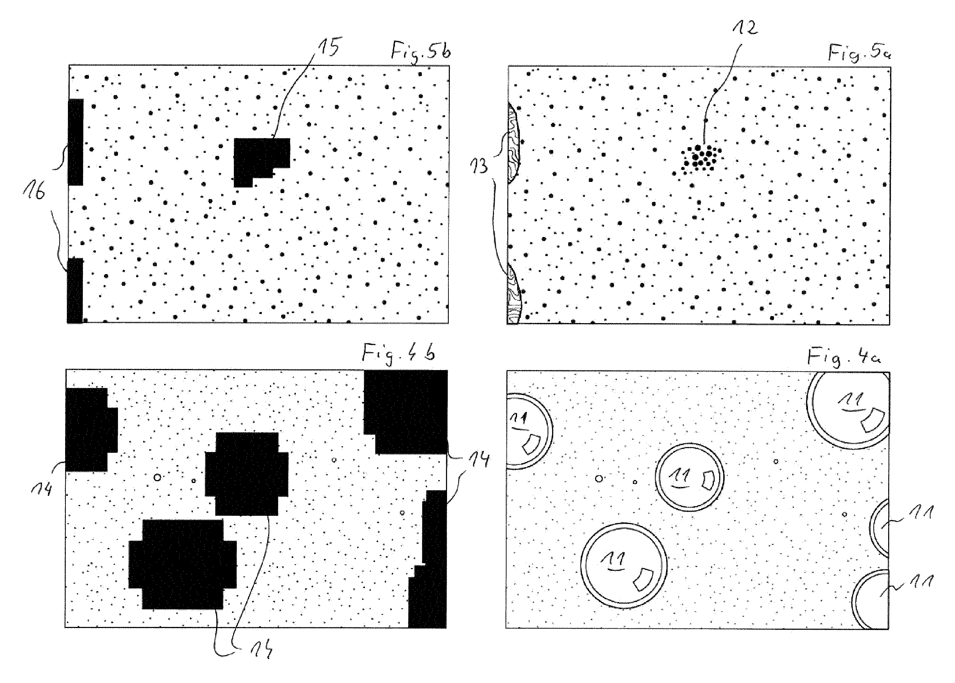

In Fig. 2a ist an der oberen linken Ecke des Bildes ein großer dunkler Fleck 3 zu erkennen, welcher das Hintergrundsignal nicht tangiert, da er vergleichsweise scharf abgegrenzt ist. Es handelt sich hierbei um einen Fleck 3, wie er typischerweise von einer Luftblase in der Flüssigkeit erzeugt wird. Dieser dunkle Fleck 3 ist in Fig. 2b durch den flachen Peak 4 neben dem Peak 2 repräsentiert. Wie sowohl anhand von Fig. 2a als auch anhand des Diagramms von Fig. 2b erkennbar ist, hat dieser vergleichsweise scharf abgegrenzte dunkle Fleck 3 praktisch keinen Einfluss auf das Hintergrundsignal. Ungeachtet dessen, wird dieser Bereich, wie weiter unten noch anhand der Fig. 4 und 5 erläutert werden wird, bei der Signalauswertung ausgeschlossen.

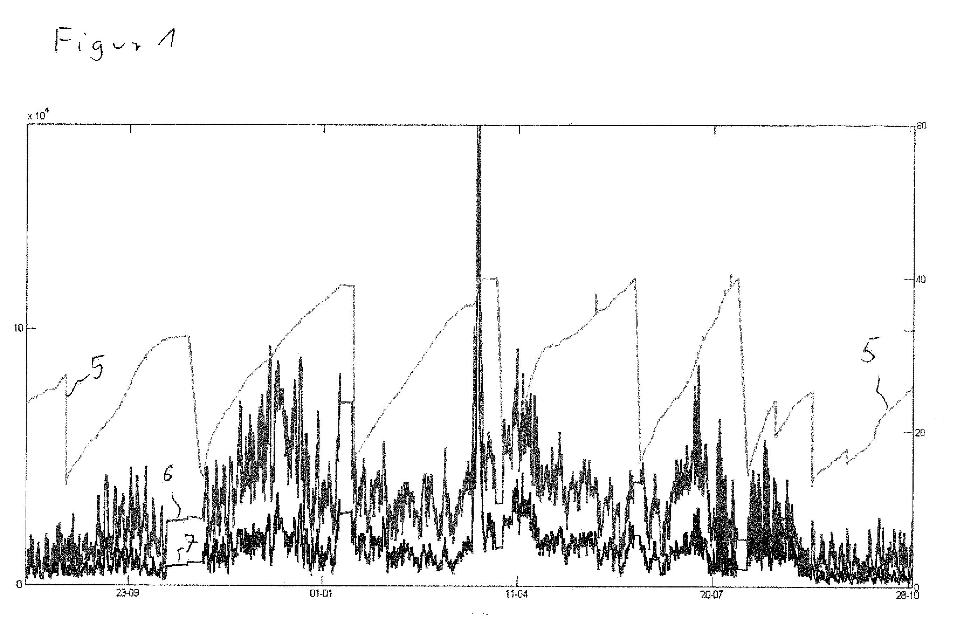

Das Hintergrundsignal, welches für jedes Bild einer Flüssigkeitsprobe, die mit einer Vielzahl von Bildern analysiert wird, ermittelt werden kann, wird aus einer Vielzahl solcher Bilder derselben Probe gebildet, zum Beispiel als Durchschnittswert von 500 Bildern und gespeichert. Dabei wird dieses Hintergrundsignal nicht nur zur Bildauswertung herangezogen, sondern auch über die Zeit registriert, wie dies anhand von Fig. 1 dargestellt ist. Der zeitliche Verlauf des Hintergrundsignals ist mit 5 gekennzeichnet, wobei die horizontale Achse den zeitlichen Verlauf (in Fig. 1 über etwa ein Jahr) und die vertikale (rechte) Achse den Pixelwert angibt, der dem Hintergrundsignal zugrundegelegt worden ist.

Das Diagramm gemäß Fig. 1 zeigt das Hintergrundsignal einer Vorrichtung zur Trinkwasserüberwachung, bei der ein auswechselbarer Probenträger vorgesehen ist, der in vorgegebenen zeitlichen Abständen von z. B. einer Stunde mit dem zu prüfenden Trinkwasser durchspült und dann im quasi stationären Zustand, wenn Zu- und Ablauf des Probenträgers verschlossen sind, untersucht wird. Dabei bildet sich zumindest im Bereich des Fensters des Probenträgers im Laufe der Zeit typischerweise ein Biofilm, welcher die optische Analyse erschwert bzw. ab einem gewissen Grad die Partikelerfassung nicht mehr mit der erforderlichen Zuverlässigkeit und Genauigkeit zulässt. Dann muss der Probenträger gewechselt oder zumindest gereinigt werden.

In dem Diagramm nach Fig. 4 liegt der maximal zulässige Pixelwert für das Hintergrundsignal bei 40. Wenn dieser Wert als Hintergrundsignal erreicht ist, muss der Probenträger ausgetauscht oder gereinigt werden. In der Figur 1 ist deutlich sichtbar, dass an vier Stellen dieser vorbestimmte Maximalwert von 40 erreicht worden ist, wonach dann der Probenträger gewechselt worden ist und das Hintergrundsignal wieder unter den Wert 20 gefallen ist. Diese vier Zeitpunkte sind etwa bei 01-01 (steht für 1. Januar), bei 01-04 (steht für 1. April), bei 01-06 (steht für 1. Juni) und bei 01-08 (steht für 1. August) erreicht. Der Pixelwert 40 stellt hier den vorbestimmten Grenzwert für das Hintergrundsignal dar, bei dem das Verfahren unterbrochen wird, zumindest die Auswertung unterbrochen wird.

Weiterhin kann anhand der Steilheit der Kurve 5 in den ansteigenden Bereichen die Geschwindigkeit der Verschmutzung ermittelt werden und damit einigermaßen zuverlässig prognostiziert werden, wann der nächste erforderliche Wechsel des Probenträgers zu erwarten sein wird. Schließlich kann ein vorbestimmter Grenzwert unterhalb dieses oberen Grenzwertes gewählt werden, der dem Anwender signalisiert, dass die Verschmutzung des Fensters einen Wert erreicht hat, der einen Wechsel des Probenträgers in absehbarer Zeit erforderlich machen wird, und diesen somit rechtzeitig ankündigen.

In der Fig. 1 ist jedoch nicht nur der zeitliche Verlauf des Hintergrundsignals 5 aufgetragen, sondern auch die Häufigkeitsverteilung der erfassten Partikel, und zwar in der Kurve 6, die die als Bakterien klassifizierten Partikel umfasst und in der Kurve 7, die die als Nicht-Bakterien klassifizierten Partikel umfasst. Wie eine solche Klassifizierung erfolgt, ist nicht Gegenstand der vorliegenden Anmeldung und daher hier nicht im Weiteren erwähnt. Dabei ist in diesen Kurven 6 und 7 zwar dieselbe horizontale Zeitachse wie der Kurve 5 zugeordnet, jedoch ist die vertikale Achse die Häufigkeitsverteilung auf der linken Skala des Diagramms angegeben. Insbesondere wie die maximal auftretende Häufigkeit von 20 x 10⁴ in der Mitte der Kurve 6 verdeutlicht, geht dieser steile Anstieg der Bakterienmenge innerhalb der Flüssigkeitsprobe einher mit einem ebenfalls steilen Anstieg des Hintergrundsignals, das heißt des sich dann bildenden Foulingfilms im Fenster. Im Übrigen zeigt das Diagramm jedoch, dass diese Schwankungen im Allgemeinen keinen Einfluss auf die Belagbildung innerhalb des Fensters haben.

Fig. 3a zeigt ein Bild, bei welchem der Verschmutzungsgrad des Probenträgerfensters deutlich angestiegen ist, so sind die schwarzen Bereiche 8 und die schlierenförmigen Bereiche 9 durch Ablagerungen gebildet, die den Messvorgang, das heißt das quantitative und qualitative Erfassen der im Fenster befindlichen Partikel, erheblich erschwert. Wie die Häufigkeitsverteilung gemäß Fig. 3b verdeutlicht, ist der Peak 10, der sich bei diesem Bild bildet, deutlich flacher und breiter als der Peak 2, der sich bei dem Bild gemäß Fig. 2a bildet. Der sich daraus ergebende Durchschnittswert für das Hintergrundsignal ist demgemäß deutlich höher, wodurch der höhere Verschmutzungsgrad repräsentiert wird.

Um die Auswertung des Sensorsignals zu verbessern, wird nicht nur das Hintergrundsignal subtrahiert, sondern es werden im Weiteren in einzelnen Bildern einzelne Gruppen von Zellen der Zellenmatrix bei der Auswertung des Signals ausgeschlossen. Hierzu wird die Zellenmatrix des Sensors, die beispielsweise 2560 mal 1920 Zellen aufweisen kann, in 400 gleich große Untermatrizen geteilt, wobei zu jeder Untermatrix in gleicher Weise ein Hintergrundsignal gebildet wird, wie dies in der vorbeschriebenen Weise für die gesamte Matrix von Zellen zur Bildung des Hintergrundsignals erfolgt. Für diese Untermatrizen wird wiederum ein vorbestimmter Grenzwert, nämlich ein weiterer Grenzwert gesetzt, der bei einer 8 Bit Auflösung zwischen 0 und 255 liegt, und hier beispielhaft ebenfalls mit 40 gewählt wird. Wenn sich dann aufgrund von großen Partikeln, wie beispielsweise Luftblasen 11, Anhäufungen von Partikeln 12 oder Reflexionserscheinungen 13 am Rand Untermatrizen ergeben, deren Hintergrundsignal 40 überschreitet, so werden diese von der weiteren Bildauswertung ausgenommen, wie dies anhand der Fig. 4b bzw. 5b dargestellt ist. So sind dort, wo im Bild 4a die Luftblasen 11 zu sehen sind, im Feld 4b schwarze, scharf abgegrenzte Felder 14 zu sehen, welche durch eine Anzahl solcher ausgeschalteter Untermatrizen von Zellen gebildet sind. Entsprechend ist in Fig. 5b mittig ein Feld 15 ausgespart, dort wo in Fig. 5a die Anhäufung von Partikeln 12 angeordnet ist, sowie Felder 16, dort wo in Fig. 5a die Reflexionserscheinungen 13 am Rand zu sehen sind. Durch diese Auswertung kann gleichzeitig eine Grobcharakterisierung größerer Partikel hinsichtlich der Größe erfolgen. Auch kann im Weiteren festgelegt werden, dass bei einer maximalen Zahl von ausgeblendeten Feldern 14, 15, 16 eine weitere Bildauswertung nicht erfolgt, um sicherzustellen, dass die Auswertungen stets ausreichend statistisch abgesichert sind.

### Bezugszeichenliste

- 1: - Pixelwert
- 2: - Peak in Fig. 2b
- 3: - dunkler Fleck in Fig. 2a
- 4: - flacher Peak in Fig. 2b
- 5: - zeitlicher Verlauf des Hintergrundsignals in Fig. 1
- 6: - Kurve, welche die Häufigkeitsverteilung der Bakterien darstellt
- 7: - Kurve, welche die Häufigkeitsverteilung der Nichtbakterien darstellt
- 8: - schwarzer Bereich in Fig. 3a
- 9: - schlierenförmiger Bereich in Fig. 3a
- 10: - Peak in Fig. 3b
- 11: - Luftblasen in Fig. 4a
- 12: - Anhäufung von Partikeln in Fig. 5a
- 13: - Reflexionserscheinungen in Fig. 5a
- 14: - ausgeblendete Felder in Fig. 4b
- 15: - ausgeblendetes Feld in Fig. 5b
- 16: - ausgeblendete Felder in Fig. 5b

## Patentansprüche

1. Verfahren zum quantitativen und/oder qualitativen Erfassen von Partikeln in Flüssigkeit, bei dem die zu untersuchende Flüssigkeit in einen Strahlengang einer optischen Einrichtung zwischen mindestens einer Lichtquelle und einem Bilderfassungssensor mit einer Matrix von lichtempfindlichen Zellen angeordnet wird, bei dem die Pixelwerte der Zellen erfasst und die Verteilung der Pixelwerte zumindest teilweise ermittelt wird, und bei dem der oder die Pixelwerte, die am häufigsten ermittelt worden sind als Wert oder Durchschnittswert für ein Hintergrundsignal verwendet werden, **dadurch gekennzeichnet, dass** beim Erreichen eines vorbestimmten Grenzwertes für das Hintergrundsignal ein Signal abgegeben oder das Verfahren unterbrochen wird.

2. Verfahren nach Anspruch 1, **dadurch gekennzeichnet dass** bei Erreichen eines ersten vorbestimmten Grenzwerts für das Hintergrundsignal ein Signal abgegeben und bei Erreichen eines zweiten vorbestimmten Grenzwerts das Verfahren unterbrochen wird.

3. Verfahren nach Anspruch 1 oder 2, **dadurch gekennzeichnet, dass** das Hintergrundsignal durch den Mittelwert von Werten oder Durchschnittswerten mehrerer nacheinander aufgenommener Bilder, vorzugsweise von 500 bis 1500 Bildern unterschiedlicher Bildebenen gebildet wird.

4. Verfahren nach einem der vorhergehenden Ansprüche, **dadurch gekennzeichnet, dass** die Werte für das Hintergrundsignal von zeitlich nacheinander erfolgten Erfassungen von Partikeln in Flüssigkeit registriert und anhand der zeitlichen Veränderung der registrierten Werte eine Änderungsgeschwindigkeit bestimmt wird.

5. Verfahren nach einem der vorhergehenden Ansprüche, **dadurch gekennzeichnet, dass** die Werte für das Hintergrundsignal von zeitlich nacheinander erfolgten Erfassungen von Partikeln in Flüssigkeit registriert und anhand der zeitlichen Veränderung der registrierten Werte bestimmt wird, wann ein vorbestimmter Grenzwert für das Hintergrundsignal voraussichtlich erreicht sein wird.

6. Verfahren nach einem der vorhergehenden Ansprüche, **dadurch gekennzeichnet, dass** das Hintergrundsignal als Maß für die Verunreinigungen im Strahlengang herangezogen wird und dass bei Erreichen eines vorbestimmten Grenzwertes ein die zu untersuchende Flüssigkeit aufnehmender Flüssigkeitsträger gereinigt oder gewechselt wird.

7. Verfahren nach einem der vorhergehenden Ansprüche, **dadurch gekennzeichnet, dass** die Bestimmung des Hintergrundsignals vor jedem Erfassen von Partikeln oder in vorbestimmten zeitlichen Abständen erfolgt.

8. Verfahren nach einem der vorhergehenden Ansprüche, **dadurch gekennzeichnet, dass** bei der Auswertung der Pixelwerte die Zellenmatrix des Sensors in eine Vielzahl von vorzugsweise gleich großen Teilmatrizen geteilt wird und für jede Teilmatrix ein Hintergrundsignal gebildet wird, und dass die Teilmatrizen, deren Hintergrundsignal einen vorbestimmten weiteren Grenzwert übersteigt, von der weiteren Auswertung zum Erfassen der Partikel ausgenommen werden.

9. Verfahren nach einem der vorhergehenden Ansprüche, **dadurch gekennzeichnet, dass** die Verteilung der Pixelwerte verwendet wird um die Partikel größenmäßig zu kategorisieren.

10. Verfahren nach einem der vorhergehenden Ansprüche, **dadurch gekennzeichnet, dass** der Grenzwert für das Hintergrundsignal der Zellenmatrix und/oder für das Hintergrundsignal einer Teilmatrix zu 10% bis 20% eines maximalen Pixelwertes vorbestimmt wird.

11. Verfahren nach einem der vorhergehenden Ansprüche, **dadurch gekennzeichnet, dass** der Grenzwert für das Hintergrundsignal der Zellenmatrix bei einer 8-bit-Auflösung eines Pixelwertes zwischen 30 und 50, vorzugsweise zwischen 35 und 40 vorbestimmt wird.

12. Verfahren nach einem der vorhergehenden Ansprüche, **dadurch gekennzeichnet, dass** der weitere Grenzwert für das Hintergrundsignal einer Teilmatrix bei einer 8-bit-Auflösung zwischen 35 und 45 vorbestimmt wird.

13. Vorrichtung zum optischen Erfassen von Partikeln in einer Flüssigkeit mit einer Lichtquelle, einem Bilderfassungssensor mit einer Matrix von lichtempfindlichen Zellen und einem im Strahlengang dazwischen angeordneten Flüssigkeitsträger, mit einer Steuerund Auswertelektronik, die die Pixelwerte der Zellen erfasst und die Verteilung der Pixelwerte zumindest teilweise ermittelt und die dem oder den Pixelwerten, die am häufigsten ermittelt worden sind, als Wert oder Durchschnittswert für ein Hintergrundsignal verwendet, **dadurch gekennzeichnet, dass** die Steuer- und Auswertelektronik beim Erreichen eines vorbestimmten Grenzwertes für das Hintergrundsignal zur Abgabe eines Signals oder zur Unterbrechung des Erfassungsvorgangs ausgebildet ist.

14. Vorrichtung nach Anspruch 13, **dadurch gekennzeichnet, dass** die Steuer- und Auswertelektronik auch zur Ausführung des Verfahrens gemäß einem der Ansprüche 2 bis 11 ausgebildet ist.
